# DEMANDE DE BREVET EUROPEEN

(11) **EP 1 676 563 A1**
(43) Date de publication de la demande: **05.07.2006**
(21) Numéro de dépôt: 05292515.3
(22) Date de dépôt: 28.11.2005
(51) Int. Cl.: A61K 8/37, A61K 8/49, A61K 8/67, A61K 8/73, A61Q 17/04, A61Q 19/00, A61K 47/00

(54) **Emulsion E/H comprenant de l'acide ascorbique ou un dérivé**

(30) Priorité: 03.01.2005 FR 0550008
(71) Demandeur: L'ORÉAL, 75008 Paris (FR)
(72) Inventeur: Monello, Aldo, 91600 Savigny sur Orge (FR)
(74) Mandataire: Kromer, Christophe

(57) **Abrégé**

L'invention concerne une composition sous forme d'émulsion huile-dans-eau comprenant de l'acide ascorbique ou l'un de ses dérivés, au moins un gélifiant hydrophile polysaccharide, au moins un ester d'acide gras en C₁₆-C₂₂ et de sorbitan, au moins un ester d'acide gras éthoxylé et au moins un polymère comportant un monomère à groupement sulfonique.

La composition présente une bonne stabilité, notamment après 8 jours à 55 °C.

Application au soin et au maquillage des matières kératiniques.

## Description

La présente invention a pour objet une émulsion huile-dans-eau cosmétique comprenant de l'acide ascorbique, un gélifiant hydrophile polysaccharide, un ester de sorbitan, un ester gras éthoxylé et un polymère à groupement sulfonique.

Il est connu d'introduire dans des compositions cosmétiques divers actifs destinés à apporter des traitements spécifiques à la peau et/ou aux cheveux. Toutefois, certains de ces actifs présentent l'inconvénient d'être instables en milieu aqueux et de se dégrader facilement au contact de l'eau, en particulier à cause de phénomènes d'oxydation. Ils perdent ainsi rapidement leur activité au cours du temps et cette instabilité va à l'encontre de l'efficacité recherchée.

On cherche ainsi depuis longtemps à formuler l'acide ascorbique ou vitamine C, du fait de ses nombreuses propriétés bénéfiques. En particulier, l'acide ascorbique stimule la synthèse du tissu conjonctif et notamment du collagène, renforce les défenses du tissu cutané contre les agressions extérieures telles que les rayonnements ultraviolets et la pollution, compense la déficience en vitamine E de la peau, dépigmente la peau et possède une fonction anti-radicaux libres. Ces deux dernières propriétés en font un excellent candidat comme actif cosmétique ou dermatologique pour lutter contre le vieillissement de la peau ou prévenir celui-ci. Malheureusement, en raison de sa structure chimique (d'alpha-cétolactone), l'acide ascorbique est très sensible à certains paramètres de l'environnement et notamment aux phénomènes d'oxydation. Il s'ensuit donc une dégradation rapide de l'acide ascorbique formulé en présence de ces paramètres, et plus particulièrement en présence d'oxygène, de lumière, d'ions métalliques, en fonction de la température, ou encore dans certaines conditions de pH (Pharm. Acta. Helv., 1969, 44, 611-667 ; STP Pharma, 1985, 4, 281-286).

En particulier, une émulsion cosmétique contenant de l'acide ascorbique qui est dégradé présente généralement une chute importante de la viscosité, ainsi qu'un déphasage : la composition devient ainsi instable et n'est plus appropriée pour l'usage envisagé.

Le but de la présente invention est donc de disposer d'une émulsion contenant de l'acide ascorbique ou l'un de ses dérivés qui soit stable, notamment pendant 8 jours à 35 °C, voire 2 mois à 55 °C, voire pendant 2 mois à 45 °C.

L'inventeur a découvert qu'une telle composition peut être obtenue en formulant l'acide ascorbique dans une émulsion huile-dans-eau en présence d'un mélange de tensioactif particuliers, d'un gélifiant hydrophile polysaccharide et d'un polymère à groupement sulfonique.

De façon plus précise, l'invention a pour objet une composition sous forme d'émulsion huile-dans-eau comprenant de l'acide ascorbique ou l'un de ses dérivés, au moins un gélifiant hydrophile polysaccharide, au moins un ester d'acide gras en C₁₆-C₂₂ et de sorbitan, au moins un ester d'acide gras éthoxylé et au moins un polymère comportant au moins un monomère à groupement sulfonique.

L'invention a également pour objet un procédé non thérapeutique de soin ou de maquillage des matières kératiniques comprenant l'application sur les matières kératiniques de la composition définie précédemment.

La composition selon l'invention comprend de l'acide ascorbique ou l'un de ses dérivés, notamment ses sels ou l'un de ses esters. On peut citer à titre d'exemple et de façon non limitative : l'acide ascorbique et ses dérivés tels que le 5,6-di-O-diméthylsilylascorbate (vendu par la Sté Exsymol sous la référence PRO-AA), le sel de potassium du dl-alpha-tocopheryl-2l-ascorbyl-phosphate (vendu par la Sté Senju Pharmaceutical sous la référence SEPIVITAL EPC), l'ascorbyl phosphate de magnésium, l'ascorbyl phosphate de sodium (vendu par la Sté Roche sous la référence Stay-C 50) et l'ascorbyl glucoside (vendu par lan société Hayashibara).

L'acide ascorbique ou l'un de ses dérivés peut être présent dans la composition selon l'invention en une teneur allant de 0,01 % à 20 % en poids, par rapport au poids total de la composition, de préférence allant de 0,01 % à 10 % en poids, et préférentiellement allant de 0,01 % à 5% en poids.

La composition selon l'invention comprend un gélifiant hydrophile polysaccharide.

Le gélifiant hydrophile polysaccharide peut être choisi parmi les glucanes, les amidons modifiés ou non (tels que ceux issus, par exemple, de céréales comme le blé, le maïs ou le riz, de légumes comme le pois blond, de tubercules comme les pommes de terre ou le manioc), l'amylose, l'amylopectine, le glycogène, les dextranes, les celluloses et leurs dérivés (méthylcelluloses, hydroxyalkylcelluloses, hydroxyéthylcelluloses, hydroxypropylcellulose, carboxyméthyl-celluloses), les mannanes, les xylanes, les lignines, les arabanes, les galactanes, les galacturonanes, la chitine, les chitosanes, les glucoronoxylanes, les arabinoxylanes, les xyloglucanes, les glucomannanes, les acides pectiques et les pectines, l'acide alginique et les alginates, les arabinogalactanes, les carraghénines, les agars, les glycosaminoglucanes, les gommes arabiques, les gommes Tragacanthe, les gommes Ghatti, les gommes Karaya, les gommes de caroube, les galactomannanes telles que les gommes de guar et leurs dérivés non ioniques (hydroxypropyl guar) et les gommes de xanthane, et leurs mélanges.

D'une manière générale, les composés de ce type, utilisables dans la présente invention, sont choisis parmi ceux qui sont notamment décrits dans "Encyclopedia of Chemical Technology, Kirk-Othmer, Third Edition, 1982, volume 3, pp. 896-900, et volume 15, pp 439-458", dans "Polymers in Nature, par E. A. MacGREGOR et C. T. GREENWOOD, Editions John Wiley & Sons, Chapter 6, pp 240-328, 1980" et dans l'Industrial Gums - Polysaccharides and their Derivatives, Edité par Roy L. WHISTLER, Second Edition, Edition Academic Press Inc.", le contenu de ces trois ouvrages étant totalement inclus dans la présente demande à titre de référence.

On utilise de préférence, les gommes de xanthane.

Le gélifiant hydrophile polysaccharide peut être présent dans la composition selon l'invention en une teneur allant de 0,01 % à 10 % en poids, par rapport au poids total de la composition, de préférence allant de 0,1 % à 5 % en poids, et préférentiellement allant de 0,1 % à 3 % en poids.

La composition selon l'invention comprend, à titre d'émulsionnant, au moins un ester d'acide gras en C₁₆-C₂₂ et de sorbitan et au moins un ester d'acide gras éthoxylés.

Les esters d'acide gras en C₁₆-C₂₂ et de sorbitan sont formés par estérification d'au moins un acide gras comportant au moins une chaîne alkyle linéaire saturée
ou insaturée, ayant respectivement de 16 à 22 atomes de carbone, avec le sorbitol. Ces esters peuvent être notamment choisis parmi les stéarates, béhénates, arachidates, palmitates, oléates de sorbitan, et leurs mélanges. On utilise de préférence des stéarates et palmitates de sorbitan, et préférentiellement les stéarates de sorbitan.
L'ester d'acide gras en C₁₆-C₂₂ et de sorbitan présent dans la composition selon l'invention est avantageusement solide à une température inférieure ou égale à 45°C.

On peut citer à titre d'exemple d'ester de sorbitan utilisable dans composition selon l'invention, le monostéarate de sorbitan (nom CTFA : Sorbitan stearate) vendu par la société Uniqema sous la dénomination Span 60, le tristéarate de sorbitan vendu par la société Uniqema sous la dénomination Span 65 V, le monopalmitate de sorbitan (nom CTFA : Sorbitan palmitate) vendu par la société Uniqema sous la dénomination Span 40, le monoléate de sorbitan vendu par la société Uniqema sous la dénomination Span 80 V , le trioléate de sorbitan vendu par la société Uniquema sous la dénomination Span 85 V.

L'ester d'acide gras en C₁₆-C₂₂ et de sorbitan peut être présent dans la composition selon l'invention en une teneur allant de 0,01 % à 10 % en poids, par rapport au poids total de la composition, et de préférence allant de 0,01 % à 5 % en poids.

L'ester gras éthoxylé présent dans la composition selon l'invention est de préférence un ester d'acides gras en C₁₆-C₂₂ comportant de 8 à 100 unités d'oxyde éthylène.

La chaîne grasse des esters peut être notamment choisie parmi les motifs stéaryle, béhényle, arachidyle, palmityle, cetyle et leurs mélanges tel que cétéaryle.

Le nombre d'unités d'oxyde d'éthylène peut aller de 8 à 100, de préférence de 10 à 80, et mieux de 20 à 60. Selon un mode particulier de réalisation de l'invention, ce nombre est de 40.

A titre d'exemple d'ester gras éthoxylé, on peut citer les esters d'acide stéarique comprenant respectivement 20, 30, 40, 50, 100 unités d'oxyde d'éthylène, tels que les produit commercialisés respectivement sous la dénomination Myrj 49 P, Myrj 51, Myrj 52 P (stéarate de polyéthylèneglycol 40 OE ; nom CTFA : PEG-40 stéarate), Myrj 53, Myrj 59 P par la société UNIQEMA,

L'ester gras éthoxylé peut être présent dans la composition selon l'invention en une teneur allant de 0,01 % à 10 % en poids, par rapport au poids total de la composition, et de préférence allant de 0,01 % à 5 % en poids.

La composition selon l'invention comprend également un polymère comportant au moins un monomère à groupement sulfonique. La présence de ce polymère permet d'obtenir une composition présentant de bonnes propriétés cosmétiques, en particulier de non filant notamment lors de la prise au doigt de la composition, de non collant, de douceur, et favorise également une bonne pénétration de la composition lors de son application sur la peau.

Les polymères comportant au moins un monomère à groupement sulfonique, utilisés dans la composition de l'invention, sont hydrosolubles ou hydrodispersibles ou gonflables dans l'eau. Les polymères utilisés conformément à l'invention peuvent être des homopolymères ou des copolymères et sont susceptibles d'être obtenus à partir d'au moins un monomère à insaturation éthylénique et à groupement sulfonique, pouvant être sous forme libre ou partiellement ou totalement neutralisée. Ces polymères peuvent éventuellement comprendre au moins un groupement hydrophobe et constituer alors un polymère amphiphile (ou polymère modifié hydrophobe).

De façon préférentielle, les polymères conformes à l'invention peuvent être neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés. Ils sont généralement neutralisés. On entend dans la présente invention par « neutralisés », des polymères totalement ou pratiquement totalement neutralisés, c'est-à-dire neutralisés à au moins 90 %.

Les polymères utilisés dans la composition de l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Ces polymères selon l'invention peuvent être réticulés ou non réticulés.

Les monomères à groupement sulfonique du polymère utilisé dans la composition de l'invention sont choisis notamment parmi l'acide vinylsulfonique, l'acide styrènesulfonique, les acides (méth)acrylamido-(C₁-C₂₂)alkylsulfoniques, les acides N-(C₁-C₂₂)alkyl-(méth)acrylamido-(C₁-C₂₂)-alkylsulfoniques comme l'acide undécyl-acrylamido-méthane-sulfonique, ainsi que leurs formes partiellement ou totalement neutralisées, et leurs mélanges.

Selon un mode préféré de réalisation de l'invention, les monomères à groupement sulfonique sont choisis parmi les acides (méth)acrylamido(C₁-C₂₂)alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide 2-méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique, ainsi que leurs formes partiellement ou totalement neutralisées, et leurs mélanges.

Plus particulièrement, on utilise l'acide 2-acrylamido-2-méthylpropane-sulfonique (AMPS) ainsi que ses formes partiellement ou totalement neutralisées.

Lorsque les polymères sont réticulés, les agents de réticulation peuvent être choisis parmi les composés à polyinsaturation oléfinique couramment utilisés pour la réticulation des polymères obtenus par polymérisation radicalaire.

On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Selon un mode préféré de réalisation de l'invention, l'agent de réticulation est choisi parmi le méthylène-bis-acrylamide, le méthacrylate d'allyle ou le triméthylol propane triacrylate (TMPTA). Le taux de réticulation va en général de 0,01 à 10 % en mole et plus particulièrement de 0,2 à 2 % en mole par rapport au polymère.

Lorsque les polymères utilisés sont des homopolymères, ils ne comportent que des monomères à groupement sulfonique et, s'ils sont réticulés, un ou plusieurs agents de réticulation.

Ces homopolymères sont généralement réticulés et neutralisés, et ils peuvent être obtenus selon le procédé de préparation comprenant les étapes suivantes :
(a) on disperse ou on dissout le monomère tel que l'acide 2-acrylamido 2-méthylpropane sulfonique sous forme libre dans une solution de tertio-butanol ou d'eau et de tertio-butanol ;
(b) on neutralise la solution ou la dispersion de monomère obtenue en (a) par une ou plusieurs bases minérales ou organiques, de préférence l'ammoniaque NH₃, dans une quantité permettant d'obtenir un taux de neutralisation des fonctions acides sulfoniques du polymère allant de 90 à 100% ;
(c) on ajoute à la solution ou dispersion obtenue en (b), le ou les monomères réticulants ;
(d) on effectue une polymérisation radicalaire classique en la présence d'amorceurs de radicaux libres à une température allant de 10 à 150°C ; le polymère précipitant dans la solution ou la dispersion à base de tertio-butanol.

Les homopolymères d'AMPS préférés sont en général caractérisés par le fait qu'ils comprennent, distribués de façon aléatoire :
a) de 90 à 99,9% en poids de motifs de formule générale (II) suivante : 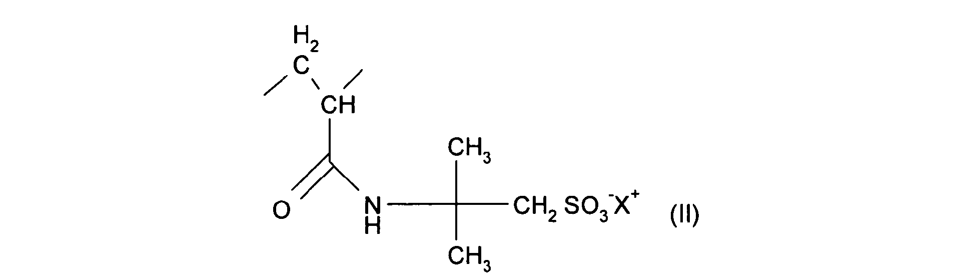 dans laquelle X⁺ désigne un proton, un cation de métal alcalin, un cation alcalino-terreux ou l'ion ammonium, au plus 10% mol des cations X⁺ pouvant être des protons H⁺ ;
b) de 0,01 à 10% en poids de motifs réticulants provenant d'au moins un monomère ayant au moins deux double-liaison oléfiniques ; les proportions en poids étant définis par rapport au poids total du polymère.

Les homopolymères selon l'invention plus particulièrement préférés comprennent de 98 à 99,5 % en poids de motifs de formule (II) et de 0,2 à 2 % en poids de motifs réticulants.

Comme polymères de ce type, on peut citer notamment l'homopolymère réticulé et neutralisé d'acide 2-acrylamido 2-méthylpropane sulfonique, commercialisé par la société Clariant sous la dénomination commerciale « Hostacerin AMPS » (nom CTFA : ammonium polyacryldimethyltauramide).

Le polymère peut être aussi un homopolymère amphiphile (ou homopolymère modifié hydrophobe) choisi parmi les polymères amphiphiles statistiques d'AMPS modifiés par réaction avec une n-monoalkylamine ou une di-n-alkylamine en C₆₋C₂₂, tels que ceux décrits dans le document WO-A-00/31154, qui sont des homopolymères greffés.

Lorsque les polymères utilisés sont des copolymères, ils sont susceptibles d'être obtenus à partir de des monomères à insaturation éthylénique et à groupement sulfonique et d'autres monomères à insaturation éthylénique, c'est-à-dire de monomères à insaturation éthylénique sans groupement sulfonique.

Les monomères à insaturation éthylénique et à groupement sulfonique sont choisis parmi ceux décrits ci-dessus.

Les monomères à insaturation éthylénique sans groupement sulfonique peuvent être choisis parmi les monomères hydrophiles à insaturation éthylénique, les monomères hydrophobes à insaturation éthylénique et leurs mélanges. Quand le polymère contient des monomères hydrophobes, il constitue un polymère amphiphile (appelé aussi polymère modifié hydrophobe).

Les monomères hydrophiles à insaturation éthylénique peuvent être choisis par exemple parmi les acides (méth)acryliques, leurs dérivés alkyl substitués en β ou leurs esters obtenus avec des monoalcools ou des mono- ou poly-alkylèneglycols, les (méth)acrylamides, la vinylpyrrolidone, le vinylformamide, l'anhydride maléique, l'acide itaconique, l'acide maléique ou les mélanges de ces composés.

Quand le polymère de la composition selon l'invention est un copolymère susceptible d'être obtenu à partir de monomères à insaturation éthylénique et à groupement sulfonique et de monomères hydrophiles à insaturation éthylénique, il peut être choisi notamment parmi (1) les copolymères anioniques réticulés d'acrylamide ou méthylacrylamide et d'acide 2-acrylamido-2-méthylpropane sulfonique, notamment ceux se présentant sous la forme d'une émulsion E/H, tels que ceux commercialisés sous le nom de SEPIGEL 305 par la société Seppic (nom C.T.F.A. : Polyacrylamide / C13-14 Isoparaffin / Laureth-7), sous le nom de SIMULGEL 600 par la société Seppic (nom C.T.F.A. : Acrylamide / Sodium acryloyldimethyltaurate copolymer / Isohexadecane / Polysorbate 80), (2) les copolymères d'acide(méth)acrylique ou de (méth)acrylate et d'acide 2-acrylamido-2-méthylpropane sulfonique, notamment ceux se présentant sous la forme d'une émulsion E/H, tels que ceux commercialisés sous le nom de SIMULGEL NS par la société Seppic (copolymère d'acrylamido-2-methyl propane sulfonate de sodium /hydroxyethyl acrylate en émulsion inverse à 40% dans Polysorbate 60 et squalane) (nom CTFA : hydroxyethyl acrylate/sodium acryloyldimethyltaurate copolymer / squalane / polysorbate 60) ou ceux commercialisés sous le nom de SIMULGEL EG par la société Seppic (copolymère d'acide acrylique / acrylamido-2-methylpropane-sulfonique sous forme de sel de sodium en émulsion inverse à 45% dans isohexadecane / eau) (nom C.T.F.A. : Sodium Acrylate / Sodium acryloydimethyl-taurate copolymer / Isohexadecane / Polysorbate 80), et (3) les copolymères d'acide 2-acrylamido-2-méthylpropane sulfonique et de vinylpyrrolidone ou de vinylformamide, tels que les produits commercialisés sous les dénominations ARISTOFLEX AVC par la société Clariant.

Quand les monomères à groupement sulfonique sont copolymérisés avec des monomères hydrophobes à insaturation éthylénique comportant une chaîne hydrophobe, appelée aussi chaîne grasse (chaîne en C6-C50), le polymère obtenu est amphiphile, c'est-à-dire qu'il comporte à la fois une partie hydrophile et une partie hydrophobe. On appelle aussi de tels polymères, des polymères modifiés hydrophobes.

Ces polymères modifiés hydrophobes peuvent contenir en outre un ou plusieurs monomères ne comportant ni groupement sulfonique ni chaîne grasse, tels que les acides (méth)acryliques, leurs dérivés alkyl substitués en β ou leurs esters obtenus avec des monoalcools ou des mono- ou poly-alkylèneglycols, les (méth)acrylamides, la vinylpyrrolidone, le vinylformamide, l'anhydride maléique, l'acide itaconique, l'acide maléique ou les mélanges de ces composés.

Comme polymères modifiés hydrophobes, on peut utiliser notamment ceux susceptibles d'être obtenus à partir d'acide 2-acrylamido-2-méthylpropane sulfonique (AMPS) et d'au moins un monomère hydrophobe à insaturation éthylénique comportant au moins un groupement ayant de 6 à 50 atomes de carbone, plus préférentiellement de 6 à 22 atomes de carbone, encore plus préférentiellement de 6 à 18 atomes de carbone et plus particulièrement 12 à 18 atomes de carbone.

Ces polymères sont décrits notamment dans les documents EP-A-750899, US-A-5089578 et WO-A-2002/:43689, et dans les publications de Yotaro Morishima suivantes :
- « Self-assembling amphiphilic polyelectrolytes and their nanostructures - Chinese Journal of Polymer Science Vol. 18, N°40, (2000), 323-336 »;
- « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » ;
- « Solution properties of miscelle networks formed by non-ionic moieties covalently bound to an polyelectrolyte : salt effects on rheological behavior - Langmuir, 2000, Vol.16, N°12, 5324-5332 » ;
   « Stimuli responsive amphiphilic copolymers of sodium 2-(acrylamido)-2-methylpropanesulfonate and associative macromonomers - Polym. Preprint, Div. Polym. Chem. 1999, 40(2), 220-221 ».

Les monomères hydrophobes de ces polymères particuliers sont choisis de préférence parmi les acrylates, alkylacrylates, acrylamides ou alkylacrylamides de formule (III) suivante : 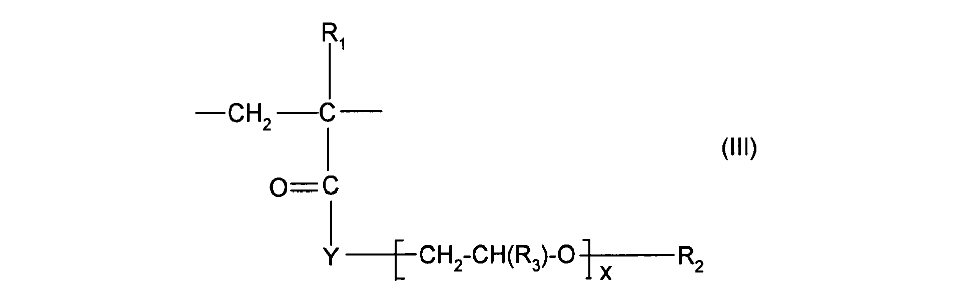 dans laquelle R₁ et R₃, identiques ou différents, désignent un atome d'hydrogène ou un radical alkyle substantiellement linéaire ou ramifié en C₁-C₆ (de préférence méthyle) ; Y désigne O ou NH ; R₂ désigne un radical hydrocarboné comportant de 6 à 50 atomes de carbone, plus préférentiellement de 6 à 22 atomes de carbone, encore plus préférentiellement de 6 à 18 atomes de carbone et plus particulièrement de 12 à 18 atomes de carbone ; x désigne un nombre de moles d'oxyde d'alkylène et varie de 0 à 100.

Le radical R₂ est choisi de préférence parmi les radicaux alkyles en C₆-C₁₈ substantiellement linéaires (par exemple n-hexyle, n-octyle, n-décyle, n-hexadécyle, n-dodécyle ou lauryle, n-octadécyle ou stéaryle), ramifiés ou cycliques (par exemple cyclododécane (C₁₂) ou adamantane (C₁₀)) ; les radicaux alkylperfluorés en C₆-C₁₈ (par exemple le groupement de formule -(CH₂)₂-(CF₂)₉₋CF₃) ; le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles substantiellement linéaires et plus particulièrement le radical n-dodécyle, n-hexadecyle ou n-octadécyle, et leurs mélanges.

Selon une forme particulièrement préférée de l'invention, le monomère de formule (III) comporte au moins un motif oxyde d'alkylène (x ≥ 1) et de préférence plusieurs motifs oxyde d'alkylène (x > 1) constituant une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés (ou nombre de moles d'oxyde d'alkylène) varie en général de 3 à 100, plus préférentiellement de 3 à 50 et encore plus préférentiellement de 7 à 25.

Parmi ces polymères, on peut citer :
- les copolymères réticulés ou non réticulés, neutralisés ou non, comportant de 15 à 60 % en poids de motifs AMPS et de 40 à 85 % en poids de motifs (C₈₋C₁₆)alkyl(méth)acrylamide ou de motifs (C₈-C₁₆)alkyl(méth)acrylate par rapport au polymère, tels que ceux décrits dans le document EP-A-750 899 ;
- les terpolymères comportant de 10 à 90% en mole de motifs acrylamide, de 0,1 à 10% en mole de motifs AMPS et de 5 à 80% en mole de motifs n-(C₆₋C₁₈)alkylacrylamide, par rapport au polymère, tels que ceux décrits dans le document US-A-5,089,578.
- les copolymères non réticulés d'AMPS partiellement ou totalement neutralisé et de méthacrylate de n-dodécyle, de n-hexadécyle ou de n-octadécyle, tels que ceux décrits dans les articles de Morishima cités ci-dessus ;
- les copolymères réticulés ou non réticulés d'AMPS partiellement ou totalement neutralisé et de n-dodécylméthacrylamide, tels que ceux décrits dans les articles de Morishima cités ci-dessus.

Comme polymères modifiés hydrophobes, on peut citer plus particulièrement les copolymères constitués (i) de motifs acide 2-acrylamido-2-méthylpropane-sulfonique (AMPS) de formule (II) indiquée ci-dessus, dans laquelle X⁺ est un proton, un cation de métal alcalin, un cation alcalino-terreux ou l'ion ammonium, et (ii) de motifs de formule (IV) suivante : 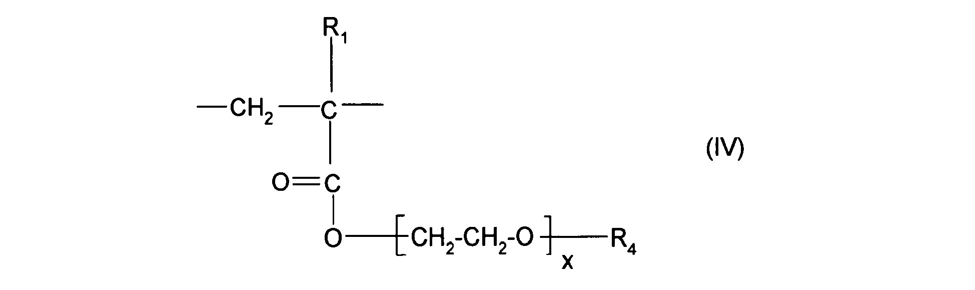 dans laquelle x désigne un nombre entier variant de 3 à 100, de préférence de 3 à 50 et plus préférentiellement de 7 à 25 ; R₁ a la même signification que celle indiquée ci-dessus dans la formule (III) et R₄ désigne un radical alkyle linéaire ou ramifié comportant de 6 à 22 atomes de carbone et de préférence de 10 à 22 atomes de carbone.

Les polymères modifiés hydrophobes de ce type sont notamment ceux décrits dans les articles de Morishima mentionnés ci-dessus, pour lesquels x = 25, R₁ désigne méthyle et R₄ représente n-dodécyle ; ou ceux décrits dans le document WO-A-02N3689, pour lesquels x = 8 ou 25, R₁ désigne méthyle et R₄ représente n-hexadécyle (C₁₆), n-octadécyle (C₁₈), ou n-dodécyle (C₁₂), ou leurs mélanges. Les polymères pour lesquels X+ désigne le sodium ou l'ammonium sont plus particulièrement préférés.

Les polymères modifiés hydrophobes préférés pouvant être utilisés dans la composition conforme à l'invention peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le chlorhydrate de 2,2-azobis-[2-amidinopropane] (ABAH = 2,2-Azo-Bis-[2-Amidinopropane] Hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Ces polymères modifiés hydrophobes peuvent être notamment obtenus par polymérisation radicalaire en milieu tert-butanol dans lequel ils précipitent. En utilisant la polymérisation par précipitation dans le tert-butanol, il est possible d'obtenir une distribution de la taille des particules du polymère particulièrement favorable pour ses utilisations.

La réaction peut être conduite à une température comprise entre 0 et 150°C, de préférence entre 10 et 100°C, soit à pression atmosphérique, soit sous pression réduite. Elle peut aussi être réalisée sous atmosphère inerte, et de préférence sous azote.

Ces polymères modifiés hydrophobes préférés sont en particulier ceux décrits dans le document EP-1,069,142, et notamment ceux obtenus par polymérisation de l'acide 2-acrylamido-2-méthylpropane-sulfonique (AMPS) ou l'un de ses sels de sodium ou d'ammonium, avec un ester de l'acide (méth)acrylique et
- d'un alcool en C₁₀-C₁₈ oxyéthyléné par 8 moles d'oxyde d'éthylène (GENAPOL® C-080 de la société CLARIANT),
- d'un alcool oxo en C₁₁ oxyéthyléné par 8 moles d'oxyde d'éthylène (GENAPOL® UD-080 de la société CLARIANT),
- d'un alcool oxo en C₁₁ oxyéthyléné par 7 moles d'oxyde d'éthylène (GENAPOL® UD-070 de la société CLARIANT),
- d'un alcool en C₁₂-C₁₄ oxyéthyléné par 7 moles d'oxyde d'éthylène (GENAPOL® LA-070 de la société CLARIANT),
- d'un alcool en C₁₂-C₁₄ oxyéthyléné par 9 moles d'oxyde d'éthylène (GENAPOL® LA-090 de la société CLARIANT),
- d'un alcool en C₁₂-C₁₄ oxyéthyléné par 11 moles d'oxyde d'éthylène (GENAPOL® LA-110 de la société CLARIANT),
- d'un alcool en C₁₆-C₁₈ oxyéthyléné par 8 moles d'oxyde d'éthylène (GENAPOL® T-080 de la société CLARIANT),
- d'un alcool en C₁₆-C₁₈ oxyéthyléné par 15 moles d'oxyde d'éthylène (GENAPOL® T-150 de la société CLARIANT),
- d'un alcool en C₁₆-C₁₈ oxyéthyléné par 11 moles d'oxyde d'éthylène (GENAPOL® T-110 de la société CLARIANT),
- d'un alcool en C₁₆-C₁₈ oxyéthyléné par 20 moles d'oxyde d'éthylène (GENAPOL® T-200 de la société CLARIANT),
- d'un alcool en C₁₆-C₁₈ oxyéthyléné par 25 moles d'oxyde d'éthylène (GENAPOL® T-250 de la société CLARIANT),
- d'un alcool en C₁₈-C₂₂ oxyéthyléné par 25 moles d'oxyde d'éthylène et/ou d'un alcool iso-C₁₆-C₁₈ oxyéthyléné par 25 moles d'oxyde d'éthylène.

La concentration molaire en % des motifs de formule (II) et des motifs de formule (IV) dans les polymères selon l'invention varie en fonction de l'application cosmétique souhaitée et des propriétés rhéologiques de la formulation recherchées. Elle peut varier entre 0,1 et 99,9 % en moles.

De préférence pour les polymères les plus hydrophobes, la proportion molaire en motifs de formule (II) ou (IV) varie de 50,1 à 99,9 %, plus particulièrement de 70 à 95 % et encore plus particulièrement de 80 à 90 %.

De préférence pour les polymères peu hydrophobes, la proportion molaire en motifs de formule (II) ou (IV) varie de 0,1 à 50 %, plus particulièrement de 5 à 25 % et encore plus particulièrement de 10 à 20 %.

La distribution des monomères dans les polymères de l'invention peut être, par exemple, alternée, bloc (y compris multibloc) ou quelconque.

Comme polymères modifiés hydrophobes de ce type, on peut citer notamment le copolymère d'AMPS et de methacrylate d'alcool en C12-C14 éthoxylé (copolymère non réticulé obtenu à partir de Genapol LA-070 et d'AMPS) (nom CTFA : Ammonium Acryloyldimethyltaurate / Laureth-7 Methacrylate Copolymer) commercialisé sous la dénomination ARISTOFLEX LNC par la société Clariant, et le copolymère d'AMPS et de méthacrylate de stéaryle éthoxylé (25 EO) (copolymère réticulé par le trimethylolpropane triacrylate, obtenu à partir de Genapol T-250 et d'AMPS) (nom CTFA : Ammonium Acryloyldimethyltaurate /Steareth-25 Methacrylate Crosspolymer) commercialisé sous la dénomination ARISTOFLEX HMS par la société Clariant.

Le polymère comportant au moins un monomère à groupement sulfonique, utilisés dans la composition conforme à l'invention, est présent en une quantité en matière active allant par exemple de 0,01 à 20 % en poids, de préférence de 0,1 à 10 % en poids, préférentiellement de 0,1 à 5 % en poids et plus préférentiellement encore de 0,5 à 3 % en poids par rapport au poids total de la composition.

La composition selon l'invention comprend une phase aqueuse.

La composition peut comprendre de l'eau en une teneur allant de 20 % à 95 % en poids, par rapport au poids total de la composition, de préférence allant de 30 % à 90 % en poids, et préférentiellement allant de 40 % à 70 % en poids.

L'eau peut être une eau florale telle que l'eau de bleuet et/ou une eau minérale telle que l'eau de VITTEL, l'eau de LUCAS ou l'eau de LA ROCHE POSAY et/ou une eau thermale.

La composition peut comprendre en outre un solvant organique miscible à l'eau à la température ambiante (25 °C) notamment choisi parmi les monoalcools ayant de 2 à 6 atomes de carbone tels que l'éthanol, l'isopropanol ;
les polyols ayant notamment de 2 à 20 atomes de carbones, de préférence ayant de 2 à 10 atomes de carbone, et préférentiellement ayant de 2 à 6 atomes de carbones, tels que la glycérine, le propylène glycol, le butylène glycol, le pentylène glycol, l'hexylène glycol, le dipropylène glycol, le diéthylène glycol ;
les éthers de glycol (ayant notamment de 3 à 16 atomes de carbone) tels que les alkyl(C₁-C₄)éther de mono, di- ou tripropylène glycol, les alkyl(C₁-C₄)éthers de mono, di- ou triéthylène glycol ; et leurs mélanges.

La composition selon l'invention peut comprendre un solvant organique miscible à l'eau à la température ambiante, notamment un polyol, en une teneur allant de 1 % à 20 % en poids, par rapport au poids total de la composition, et de préférence allant de 3 % à 15 % en poids.

Avantageusement, la composition selon l'invention a un pH allant de 3,0 à 8,0 , de préférence allant de 4,0 à 8,0, préférentiellement allant de 5,0 à 7,0, et plus préférentiellement allant de 5,5 à 6,5.

L'émulsion selon l'invention comprend également une phase huileuse.

Comme huiles plus particulièrement utilisables dans la composition de l'invention, on peut citer par exemple :
- les huiles hydrocarbonées d'origine animale, telles que le perhydrosqualène (ou squalane) ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; les esters du pentaérythritol comme le tétraisostéarate de pentaérythrytyle ; les dérivés lipophiles d'acides aminés, tels que le lauroyl sarcosinate d'isopropyle (nom INCl : Isopropyl Lauroyl sarcosinate) commercialisé sous la dénomination Eldew SL 205 par la société Ajinomoto ;
- les hydrocarbures linéaires ou ramifiés, d'origine minérale ou synthétique, tels que les huiles minérales (mélange d'huiles hydrocarbonées dérivées du pétrole ; nom INCI : Mineral oil), les huiles de paraffine, volatiles ou non, et leurs dérivés, la vaseline, les polydécènes, l'isohexadecane, l'isododecane, l'isoparaffine hydrogéné tel que l'huile de Parléam® commercialisée par la société NOF Corporation (nom INCl ; Hydrogenated Polyisobutene) ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclopentasiloxane et la cyclohexadiméthylsiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- les huiles fluorées telles que celles partiellement hydrocarbonées et/ou siliconées comme celles décrites dans le document JP-A-2-295912 ;
- les éthers tels que l'éther dicaprylique (nom CTFA : Dicaprylyl ether) ; et les benzoates d'alcools gras en C₁₂-C₁₅ (Finsolv TN de FINETEX) ;
- leurs mélanges.

L'huile peut être présente dans la composition selon l'invention en une teneur allant de 1 % à 50 % en poids, par rapport au poids total de la composition, de préférence allant de 5 % à 40 % en poids, et préférentiellement allant de 5 % à 30 % en poids.

La phase huileuse de l'émulsion peut comprendre d'autres corps gras tels que les cires ; les gommes telles que les gommes de silicone (diméthiconol) ; les résines de silicone, et leurs mélanges.

La composition selon l'invention peut comprendre au moins un agent photoprotecteur organique actif dans l'UVA et/ou l'UVB (absorbeurs), hydrosolubles ou liposolubles ou bien insolubles dans les solvants cosmétiques couramment utilisés.

La composition selon l'invention présentant une bonne stabilité, elle est appropriée pour la formulation de filtres UV organiques : les filtres UV incorporés dans la composition ne sont pas dégradés en présence de la Vitamine C.

Les filtres organiques sont notamment choisis parmi les anthranilates ; les dérivés cinnamiques ; les dérivés de dibenzoylméthane ; les dérivés salicyliques, les dérivés du camphre ; les dérivés de triazine autres que ceux de l'invention tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US 5,237,071, US 5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres organiques, on peut citer ceux désignés ci-dessous sous leur nom INCl :

**Dérivés de l'acide para-aminobenzoique :**
PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

**Dérivés salicyliques:**
Homosalate vendu sous le nom « Eusolex HMS » par Rona/EM Industries,
Ethylhexyl Salicylate vendu sous le nom « NEO HELIOPAN OS » par HAARMANN et REIMER,
Dipropyleneglycol Salicylate vendu sous le nom « DIPSAL » par SCHER,
TEA Salicylate, vendu sous le nom « NEO HELIOPAN TS » par HAARMANN et REIMER,

**Dérivés du dibenzoylméthane :**
Butyl Methoxydibenzoylmethane vendu notamment sous le nom commercial « PARSOL 1789 » par HOFFMANN LA ROCHE,
Isopropyl Dibenzoylmethane,

**Dérivés cinnamiques :**
Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
DEA Methoxycinnamate,
Diisopropyl Methylcinnamate,
Glyceryl Ethylhexanoate Dimethoxycinnamate

**Dérivés de β,β'-diphénylacrylate :**
Octocrylene vendu notamment sous le nom commercial « UVINUL N539 » par BASF,
Etocrylene, vendu notamment sous le nom commercial « UVINUL N35 » par BASF,

**Dérivés de la benzophénone :**
Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12
Diethylamino Hydroxybenzoyl Hexyl Benzoate vendu sous le nom commercial « UVINUL A PLUS» par BASF,

**Dérivés du benzylidène camphre :**
3-Benzylidene camphor fabriqué sous le nom « MEXORYL SD» par CHIMEX,
4-Methylbenzylidene camphor vendu sous le nom « EUSOLEX 6300 » par MERCK,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

**Dérivés du phenyl benzimidazole :**
Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par HAARMANN et REIMER,

**Dérivés de la triazine :**
Bis-Ethylhexyloxyphenol Methoxyphenyl Triazine vendu sous le nom commercial «TINOSORB S » par CIBA GEIGY,
Ethylhexyl triazone vendu notamment sous le nom commercial «UVINUL T150 » par BASF,
Diethylhexyl Butamido Triazone vendu sous le nom commercial « UVASORB HEB » par SIGMA 3V,

**Dérivés du phenyl benzotriazole :**
Drometrizole Trisiloxane vendu sous le nom « Silatrizole » par RHODIA CHIMIE Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

**Dérivés anthranilipues :**
Menthyl anthranilate vendu sous le nom commercial commercial « NEO HELIOPAN MA » par HAARMANN et REIMER,

**Dérivés d'imidazolines :**
Ethylhexyl Dimethoxybenzylidene Dioxoimidazoline Propionate,

**Dérivés du benzalmalonate :**
Polyorganosiloxanes à fonction benzalmalonate tels que le Polysilicone-15 vendu sous la dénomination commerciale « PARSOL SLX » par HOFFMANN LA ROCHE

**Dérivés de 4,4-diarylbutadiène :**
-1,1-dicarboxy (2,2'-diméthyl-propyl)-4,4-diphénylbutadiène

**Dérivés de benzoxazole :**
2,4-bis-[5-1 (diméthylpropyl)benzoxazol-2-yl-(4-phenyl)-imino]-6-(2-ethylhexyl)-imino-1,3,5-triazine vendu sous le nom d'Uvasorb K2A par Sigma 3V et leurs mélanges.

Les filtres UV organiques préférentiels sont choisis parmi
Ethylhexyl Salicylate,
Homosalate,
Ethylhexyl Methoxycinnamate
Butyl Methoxydibenzoylmethane
Octocrylene,
Phenylbenzimidazole Sulfonic Acid,
Disodium Phenyl Dibenzimidazole Tetra-sulfonate,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Bis-Ethylhexyloxyphenol Methoxyphenyl Triazine
Ethylhexyl triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine
et leurs mélanges.

Avantageusement, on utilise un filtre protecteur organique non ionique.

L'agent photoprotecteur peut être présent dans la composition selon l'invention en une teneur allant de 0,01 % à 30% en poids, par rapport au poids total de la composition, de préférence allant de 0,1 à 25% en poids , et préférentiellement allant de 0,1 % à 20 % en poids.

La composition selon l'invention peut comprendre en outre des charges.

Par charges, il faut comprendre des particules de toute forme, incolores ou blanches, minérales ou organiques, insolubles dans le milieu de la composition quelle que soit la température à laquelle la composition est fabriquée, et qui ne colorent pas la composition.

Les charges peuvent être de toute forme, plaquettaires, sphériques ou oblongues, quelle que soit la forme cristallographique (par exemple feuillet, cubique, hexagonale, orthorombique, etc) . On peut citer : le talc, le mica, la silice, le kaolin, de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthanes, les charges composites, les microsphères de silice creuses, et les microcapsules de verre ou de céramique.

Les charges peuvent être présentes dans la composition en une teneur allant de 0,1 % à 15 % en poids, et de préférence allant de 0,1 % à 10 % en poids, et préférentiellement de 0,1 % à 5 % en poids, par rapport au poids total de la composition.

La composition selon l'invention peut comprendre en outre un actif choisi parmi les agents desquamants capables d'agir, soit en favorisant l'exfoliation, soit sur les enzymes impliquées dans la desquamation ou la dégradation des cornéodesmosomes, les agents hydratants, les agents dépigmentants ou propigmentants, les agents anti-glycation, les inhibiteurs de NO-synthase, les inhibiteurs de 5α-réductase, les inhibiteurs de lysyl et/ou prolyl hydroxylase, les agents stimulant la synthèse de macromolécules dermiques ou épidermiques et/ou empêchant leur dégradation, les agents stimulant la prolifération des fibroblastes ou des kératinocytes et/ou la différenciation des kératinocytes, les agents myorelaxants, les agents anti-microbiens, les agents tenseurs, les agents anti-pollution ou anti-radicalaires, les agents anti-inflammatoires, les actifs lipolytiques ou ayant une activité favorable, directe ou indirecte, sur la diminution du tissu adipeux, les agents agissant sur la microcirculation, et les agents agissant sur le métabolisme énergétique des cellules.

Avantageusement, l'émulsion selon l'invention peut avoir une viscosité, mesurée à 25 °C, à une vitesse de cisaillement de 200 min⁻¹ (200 tours par minute, soit une fréquence de 50 Hz), allant de 1,5 à 2 Pa.s (15 à 20 poises), et de préférence allant de 1,7 à 1,9 Pa.s (17 à 19 poises). La viscosité est mesurée à 25 °C avec un viscosimètre RHEOMAT 180 de chez METTLER équipé d'un mobile n°3, la mesure étant effectuée après 10 minutes de rotation du mobile (temps au bout duquel on observe une stabilisation de la viscosité et de la vitesse de rotation du mobile), à un cisaillement de 200 min⁻¹.

La composition selon l'invention est en particulier destinée à un usage topique, notamment cosmétique ou dermatologique.

De façon connue, la composition cosmétique ou dermatologique de l'invention peut contenir également des adjuvants habituels dans le domaine cosmétique ou dermatologique, tels que les conservateurs, les parfums, les bactéricides, les absorbeurs d'odeur, les matières colorantes, les sels, les tensioactifs, les épaississants, les bases. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse ou dans la phase aqueuse.

La composition selon l'invention peut être appliquée sur la peau, les poils, les cils, les cheveux, les ongles ou les lèvres, suivant l'usage auquel elle est destinée. Elle peut ainsi être utilisée dans un procédé de traitement cosmétique de la peau, comprenant l'application de la composition selon l'invention sur la peau, par exemple en vue de la tonifier, de la régénérer, de lisser les ridules de la peau, et/ou pour lutter contre le vieillissement cutané, contre les méfaits des rayonnements UV et/ou pour renforcer les tissus cutanés contre les agressions de l'environnement.

En variante, la composition selon l'invention peut être utilisée pour la fabrication d'une préparation dermatologique, telle qu'une préparation destinée à dépigmenter la peau, les poils et/ou les cheveux.

La composition peut être une composition de soin, notamment être un produit de soin de la peau tels qu'une base de soin pour la peau, une crème de soin (crème de jour, de nuit, anti-rides), une base de maquillage ; une composition de soin pour les lèvres (baume à lèvres) ; une composition de protection solaire ou autobronzante.

La composition peut également être une composition de maquillage, notamment de maquillage de la peau, des lèvres, des cils, des sourcils, des cheveux. En particulier, la composition de maquillage peut être un fond de teint, un fard à joue, un fard à paupière, un produit anti-cernes, un produit de maquillage du corps.

Avantageusement, la composition est une composition non rincée.

L'émulsion selon l'invention peut être préparée selon le mode opératoire général suivant :
Mélanger les constituants de la phase aqueuse en chauffant à une température d'environ 70 °C. Mélanger par ailleurs les huiles et les tensioactifs en chauffant à une température d'environ 80 °C. Verser la phase grasse dans la phase aqueuse, à une température d'environ 70 °C puis agiter pendant 10 minutes à l'aide d'une turbine, à grande vitesse. Refroidir l'émulsion obtenue à environ 60 °C. Ajouter ensuite les épaississants, puis agiter à nouveau pendant 10 minutes. Refroidir à environ 50 °C. Introduire ensuite l'acide ascorbique ou dérivé préalablement mélangé avec de l'eau, puis les autres actifs éventuellement.

L'invention sera maintenant illustrée à l'aide des exemples non limitatifs suivants.

### Exemple 1 :

On a préparé une composition de soin du visage sous forme d'émulsion eau-dans-huile ayant la composition suivante :

| | | |
|---|---|---|
| - Tristéarate de sorbitan (SPAN 65 V d'Uniquema) | | 0,9 g |
| - Stéarate de PEG (40 OE) (MYRJ 52 P d'Uniquema) | | 2 g |
| - Alcool cétylique | | 2 g |
| - Mélange de mono, distéarate (36/64) de glycéryle | | 1,5 g |
| - Acide stéarique | | 1 g |
| - 4-tertio-butyl-4'-méthoxy-dibenzoylméthane | | 3 g |
| - 2-cyano-3,3-diphénylacrylate de 2-éthyl hexyle | | 7 g |
| - Salicylate de 2-éthyl hexyle | | 5 g |
| - Huile de vaseline | | 4 g |
| - Huile de silicone | | 10 g |
| - Acide ascorbique | | 3 g |
| - Hydroxyde de sodium | | 0,67 g |
| - Gomme de Xanthane | | 0,2 g |
| - Copolymère acrylamide/acrylamido-2-méthyl propane sulfonate de sodium en émulsion à 40 % de matière active (SIMULGEL® 600 de SEPPIC) | | 2 g |
| - Charge | | 3 g |
| - Actif antiride | | 1 g |
| - Glycérol | | 3 g |
| - Conservateurs | qs | |
| - Eau | qsp | 100 g |

Cette émulsion présente une bonne stabilité après 8 jours à 55 °C et après 2 mois à température ambiante.

La composition est appliquée sur le visage en utilisation quotidienne de jour.

### Exemple 2 comparatif :

On a préparé une composition similaire à celle de l'exemple 1 mais ne contenant pas de gomme de xanthane (la quantité de 0,2 g est remplacée par de l'eau) (composition ne faisant pas partie de l'invention).

La composition obtenue est instable après conservation pendant 3 jours à 55 °C, avec relarguage en surface de la phase huileuse.

## Revendications

1. Composition sous forme d'émulsion huile-dans-eau comprenant de l'acide ascorbique ou l'un de ses dérivés, au moins un gélifiant hydrophile polysaccharide, au moins un ester d'acide gras en C₁₆-C₂₂ et de sorbitan, au moins un ester d'acide gras éthoxylé et au moins un polymère comportant au moins un monomère à groupement sulfonique.

2. Composition selon la revendication précédente, **caractérisée par le fait que** les dérivés de l'acide ascorbique sont choisis parmi le 5,6-di-O-diméthylsilylascorbate, le sel de potassium du dl-alpha-tocopheryl-2l-ascorbyl-phosphate, l'ascorbyl phosphate de magnésium, l'ascorbyl phosphate de sodium, l'ascorbyl glucoside.

3. Composition selon l'une quelconque des revendications précédentes, **caractérisée par le fait que** la composition comprend de l'acide ascorbique.

4. Composition selon la revendication précédente, **caractérisée par le fait que** l'acide ascorbique ou l'un de ses dérivés est présent en une teneur allant de 0,01 % à 20 % en poids, par rapport au poids total de la composition, de préférence allant de 0,01 % à 10 % en poids, et préférentiellement allant de 0,01 % à 5% en poids.

5. Composition selon l'une quelconque des revendications précédentes, **caractérisée par le fait que** le gélifiant hydrophile polysaccharide est choisi parmi les glucanes, les amidons modifiés ou non, l'amylose, l'amylopectine, le glycogène, les dextranes, les celluloses et leurs dérivés, les mannanes, les xylanes, les lignines, les arabanes, les galactanes, les galacturonanes, la chitine, les chitosanes, les glucoronoxylanes, les arabinoxylanes, les xyloglucanes, les glucomannanes, les acides pectiques et les pectines, l'acide alginique et les alginates, les arabinogalactanes, les carraghénines, les agars, les glycosaminoglucanes, les gommes arabiques, les gommes Tragacanthe, les gommes Ghatti, les gommes Karaya, les gommes de caroube, les galactomannanes telles que les gommes de guar et leurs dérivés non ionique, les gommes de xanthane, et leurs mélanges.

6. Composition selon l'une quelconque des revendications précédentes, **caractérisée par le fait que** le gélifiant hydrophile polysaccharide est choisi parmi les gommes de xanthane.

7. Composition selon l'une quelconque des revendications précédentes, **caractérisée par le fait que** le gélifiant hydrophile polysaccharide est présent en une teneur allant de 0,01 % à 10 % en poids, par rapport au poids total de la composition, de préférence allant de 0,1 % à 5 % en poids, et préférentiellement allant de 0,1 % à 3 % en poids.

8. Composition selon l'une quelconque des revendications précédentes, **caractérisée par le fait que** l'ester d'acide gras en C₁₆-C₂₂ et de sorbitan est choisi parmi les stéarates de sorbitan, les béhénates de sorbitan, les arachidates de sorbitan, les palmitates de sorbitan, les oléates de sorbitan et leurs mélanges.

9. Composition selon l'une quelconque des revendications précédentes, **caractérisée par le fait que** l'ester d'acide gras en C₁₆-C₂₂ et de sorbitan est choisi parmi les stéarates de sorbitan, les palmitates de sorbitan.

10. Composition selon l'une quelconque des revendications précédentes, **caractérisée par le fait que** l'ester d'acide gras en C₁₆-C₂₂ et de sorbitan est choisi parmi les stéarates de sorbitan.

11. Composition selon l'une quelconque des revendications précédentes, **caractérisée par le fait que** l'ester d'acide gras en C₁₆-C₂₂ et de sorbitan est choisi parmi le monostéarate de sorbitan, le tristéarate de sorbitan.

12. Composition selon l'une quelconque des revendications précédentes, **caractérisée par le fait que** l'ester d'acide gras en C₁₆-C₂₂ et de sorbitan est présent en une teneur allant de 0,01 % à 10 % en poids, par rapport au poids total de la composition, et de préférence allant de 0,01 % à 5 % en poids.

13. Composition selon l'une quelconque des revendications précédentes, **caractérisée par le fait que** l'ester gras éthoxylé est un ester d'acides gras en C₁₆-C₂₂ comportant de 8 à 100 unités d'oxyde éthylène.

14. Composition selon l'une quelconque des revendications précédentes, **caractérisée par le fait que** l'ester gras éthoxylé est un ester d'acides gras en C₁₆-C₂₂ comportant de 10 à 80 unités d'oxyde d'éthylène.

15. Composition selon l'une quelconque des revendications précédentes, **caractérisée par le fait que** l'ester gras éthoxylé est un ester d'acides gras en C₁₆-C₂₂ comportant 40 unités d'oxyde d'éthylène.

16. Composition selon l'une quelconque des revendications précédentes, **caractérisée par le fait que** l'ester gras éthoxylé est le stéarate de polyéthylèneglycol 40 OE.

17. Composition selon l'une quelconque des revendications précédentes, **caractérisée par le fait que** l'ester gras éthoxylé est présent en une teneur allant de 0,01 % à 10 % en poids, par rapport au poids total de la composition, et de préférence allant de 0,01 % à 5 % en poids.

18. Composition selon l'une quelconque des revendications précédentes, **caractérisée par le fait que** le monomère à groupement sulfonique est choisi parmi l'acide vinylsulfonique, l'acide styrènesulfonique, les acides (méth)acrylamido(C₁₋C₂₂)alkylsulfoniques, les acides N-(C₁-C₂₂)alkyl-(méth)acrylamido(C₁-C₂₂)alkyl-sulfoniques, ainsi que leurs formes partiellement ou totalement neutralisées, et leurs mélanges.

19. Composition selon l'une quelconque des revendications précédentes, **caractérisée par le fait que** le monomère à groupement sulfonique est l'acide 2-acrylamido 2-méthylpropane sulfonique.

20. Composition selon l'une quelconque des revendications précédentes, **caractérisée par le fait que** le polymère est un homopolymère réticulé et neutralisé d'acide 2-acrylamido 2-méthylpropane sulfonique.

21. Composition selon l'une quelconque des revendications précédentes, **caractérisée par le fait que** le polymère comportant au moins un monomère à groupement sulfonique est un homopolymère amphiphile choisi parmi les polymères amphiphiles statistiques d'acide 2-acrylamido 2-méthylpropane sulfonique modifiés par réaction avec une n-monoalkylamine ou une di-n-alkylamine en C₆-C₂₂.

22. Composition selon l'une quelconque des revendications précédentes, **caractérisée par le fait que** le polymère comportant au moins un monomère à groupement sulfonique est choisi parmi les copolymères anioniques réticulés d'acrylamide ou méthylacrylamide et d'acide 2-acrylamido-2-méthylpropane sulfonique, les copolymères d'acide(méth)acrylique ou de (méth)acrylate et d'acide 2-acrylamido-2-méthylpropane sulfonique, et les copolymères d'acide 2-acrylamido-2-méthylpropane sulfonique et de vinylpyrrolidone ou de vinylformamide.

23. Composition selon l'une quelconque des revendications 1 à 17, **caractérisée par le fait que** le polymère comportant au moins un monomère à groupement sulfonique est un polymère modifié hydrophobe susceptible d'être obtenu à partir d'acide 2-acrylamido 2-méthylpropane sulfonique et d'au moins un monomère hydrophobe à insaturation éthylénique comportant au moins un groupement ayant de 6 à 50 atomes de carbone, de préférence de 6 à 22 atomes de carbone.

24. Composition selon la revendication précédente, **caractérisée par le fait que** le polymère modifié hydrophobe est choisi parmi :
- les copolymères réticulés ou non réticulés, neutralisés ou non, comportant de 15 à 60% en poids de motifs AMPS et de 40 à 85% en poids de motifs (C₈₋C₁₆)alkyl(méth)acrylamide ou de motifs (C₈-C₁₆)alkyl(meth)acrylate, par rapport au polymère ;
- les terpolymères comportant de 10 à 90% en mole de motifs acrylamide, de 0,1 à 10% en mole de motifs AMPS et de 5 à 80% en mole de motifs n-(C₆₋C₁₈)alkylacrylamide, par rapport au polymère ;
- les copolymères non réticulés d'AMPS partiellement ou totalement neutralisé et de méthacrylate de n-dodécyle, de n-hexadécyle ou de n-octadécyle ;
- les copolymères réticulés ou non réticulés d'AMPS partiellement ou totalement neutralisé et de n-dodécylméthacrylamide.

25. Composition selon la revendication 23, **caractérisée par le fait que** le polymère modifié hydrophobe est choisi parmi les copolymères constitués de motifs acide 2-acrylamido-2-méthylpropane-sulfonique de formule (II) : 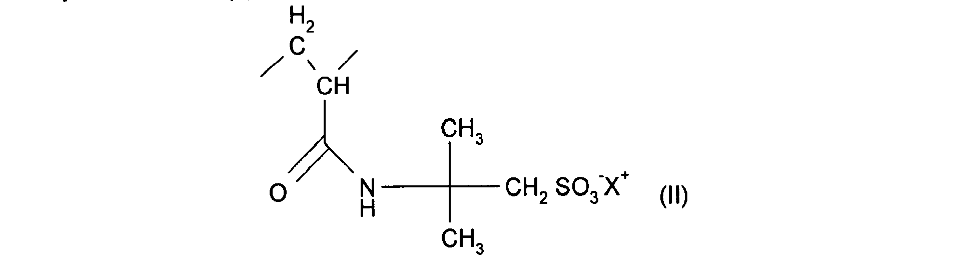 dans laquelle X⁺ est un proton, un cation de métal alcalin, un cation alcalino-terreux ou l'ion ammonium, et
(ii) de motifs de formule (IV) suivante : 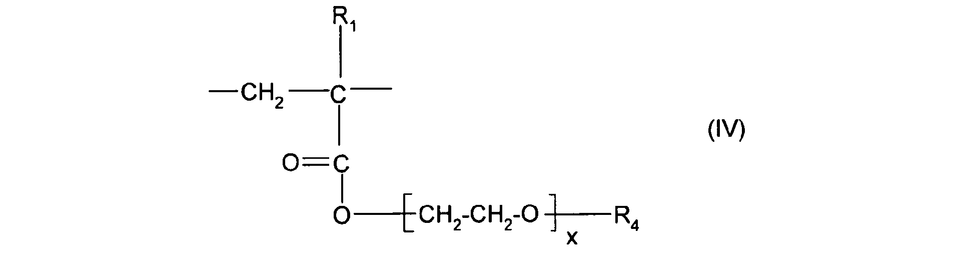 dans laquelle x désigne un nombre entier variant de 3 à 100, de préférence de 5 à 80 et plus préférentiellement de 7 à 25 ; R₁ a la même signification que celle indiquée ci-dessus dans la formule (III) et R₄ désigne un radical alkyle linéaire ou ramifié comportant de 6 à 22 atomes de carbone et de préférence de 10 à 22 atomes de carbone.

26. Composition selon l'une quelconque des revendications précédentes, **caractérisée par le fait que** le polymère comportant au moins un monomère à groupement sulfonique est présent en une quantité en matière active allant de 0,01 à 20 % en poids, par rapport au poids total de la composition, de préférence de 0,1 à 10 % en poids, préférentiellement de 0,1 à 5 % en poids et plus préférentiellement encore de 0,5 à 3 % en poids.

27. Composition selon l'une quelconque des revendications précédentes, **caractérisée par le fait qu'**elle comprend de l'eau en une teneur allant de 20 % à 95 % en poids, par rapport au poids total de la composition, de préférence allant de 30 % à 90 % en poids, et préférentiellement allant de 40 % à 70 % en poids.

28. Composition selon l'une quelconque des revendications précédentes, **caractérisée par le fait qu'**elle comprend un solvant organique miscible à l'eau à la température ambiante.

29. Composition selon la revendication précédente, **caractérisée par le fait que** le solvant organique miscible à l'eau à la température ambiante est choisi parmi les monoalcools ayant de 2 à 6 atomes de carbone, les polyols ayant de 2 à 20 atomes de carbones, les alkyl(C₁-C₄)éther de mono, di- ou tripropylène glycol, les alkyl(C₁-C₄)éthers de mono, di- ou triéthylène glycol, et leurs mélanges.

30. Composition selon la revendication 28 ou 29, **caractérisée par le fait que** le solvant organique miscible à l'eau à la température ambiante est choisi parmi l'éthanol, l'isopropanol, la glycérine, le propylène glycol, le butylène glycol, le pentylène glycol, l'hexylène glycol, le dipropylène glycol, le diéthylène glycol, et leurs mélanges.

31. Composition selon l'une quelconque des revendications 28 à 30, **caractérisée par** le fait le un solvant organique miscible à l'eau à température ambiante est présent en une teneur allant de 1 % à 20 % en poids, par rapport au poids total de la composition, et de préférence allant de 3 % à 15 % en poids.

32. Composition selon l'une quelconque des revendications précédentes, **caractérisée par le fait qu'**elle a un pH allant de 3,0 à 8,0 , de préférence allant de 4,0 à 8,0, préférentiellement allant de 5,0 à 7,0, et plus préférentiellement allant de 5,5 à 6,5.

33. Composition selon l'une quelconque des revendications précédentes, **caractérisée par le fait qu'**elle comprend une huile en une teneur allant de 1 % à 50 % en poids, par rapport au poids total de la composition, de préférence allant de 5 % à 40 % en poids, et préférentiellement allant de 5 % à 30 % en poids.

34. Composition selon l'une quelconque des revendications précédentes, **caractérisée par le fait qu'**elle comprend au moins un agent photoprotecteur organique actif dans l'UVA et/ou l'UVB.

35. Composition selon la revendication précédente, **caractérisée par le fait que** l'agent photoprotecteur est présent en une teneur allant de 0,01 % à 30% en poids, par rapport au poids total de la composition, de préférence allant de 0,1 à 25% en poids , et préférentiellement allant de 0,1 % à 20 % en poids.

36. Composition selon l'une quelconque des revendications précédentes, **caractérisée par le fait qu'**elle a une viscosité, mesurée à 25 °C, à une vitesse de cisaillement de 200 min⁻¹, allant de 1,5 à 2 Pa.s, et de préférence allant de 1,7 à 1,9 Pa.s.

37. Composition selon l'une quelconque des revendications précédentes, **caractérisée par le fait qu'**elle est une composition cosmétique ou dermatologique.

38. Composition selon l'une quelconque des revendications précédentes, **caractérisée par le fait qu'**elle comprend au moins un adjuvant cosmétique ou dermatologique choisi parmi les charges, les conservateurs, les parfums, les bactéricides, les absorbeurs d'odeur, les matières colorantes, les sels, les tensioactifs, les épaississants, les bases.

39. Procédé non thérapeutique de soin ou de maquillage des matières kératiniques comprenant l'application sur les matières kératiniques d'une composition selon l'une quelconque des revendications précédentes.

40. Procédé de traitement cosmétique de la peau, comprenant l'application sur la peau d'une composition selon l'une quelconque des revendications 1 à 38, en vue de la tonifier, de la régénérer, de lisser les ridules de la peau, et/ou pour lutter contre le vieillissement cutané, contre les méfaits des rayonnements UV et/ou pour renforcer les tissus cutanés contre les agressions de l'environnement.

## Revendications modifiées

### Revendications modifiées conformément à la règle 86(2) CBE.

**1.** Composition sous forme d'émulsion huile-dans-eau comprenant de l'acide ascorbique ou l'un de ses dérivés choisi parmi ses sels, ses esters et l'ascorbyl glucoside, au moins un gélifiant hydrophile polysaccharide, au moins un ester d'acide gras en C₁₆-C₂₂ et de sorbitan, au moins un ester d'acide gras éthoxylé et au moins un polymère comportant au moins un monomère à groupement sulfonique.

**2.** Composition selon la revendication précédente, **caractérisée par le fait que** les dérivés de l'acide ascorbique sont choisis parmi le 5,6-di-O-diméthylsilylascorbate, le sel de potassium du dl-alpha-tocopheryl-2l-ascorbyl-phosphate, l'ascorbyl phosphate de magnésium, l'ascorbyl phosphate de sodium, l'ascorbyl glucoside.

**3.** Composition selon l'une quelconque des revendications précédentes, **caractérisée par le fait que** la composition comprend de l'acide ascorbique.

**4.** Composition selon la revendication précédente, **caractérisée par le fait que** l'acide ascorbique ou l'un de ses dérivés est présent en une teneur allant de 0,01 % à 20 % en poids, par rapport au poids total de la composition, de préférence allant de 0,01 % à 10 % en poids, et préférentiellement allant de 0,01 % à 5% en poids.

**5.** Composition selon l'une quelconque des revendications précédentes, **caractérisée par le fait que** le gélifiant hydrophile polysaccharide est choisi parmi les glucanes, les amidons modifiés ou non, l'amylose, l'amylopectine, le glycogène, les dextranes, les celluloses et leurs dérivés, les mannanes, les xylanes, les lignines, les arabanes, les galactanes, les galacturonanes, la chitine, les chitosanes, les glucoronoxylanes, les arabinoxylanes, les xyloglucanes, les glucomannanes, les acides pectiques et les pectines, l'acide alginique et les alginates, les arabinogalactanes, les carraghénines, les agars, les glycosaminoglucanes, les gommes arabiques, les gommes Tragacanthe, les gommes Ghatti, les gommes Karaya, les gommes de caroube, les galactomannanes telles que les gommes de guar et leurs dérivés non ionique, les gommes de xanthane, et leurs mélanges.

**6.** Composition selon l'une quelconque des revendications précédentes, **caractérisée par le fait que** le gélifiant hydrophile polysaccharide est choisi parmi les gommes de xanthane.

**7.** Composition selon l'une quelconque des revendications précédentes, **caractérisée par le fait que** le gélifiant hydrophile polysaccharide est présent en une teneur allant de 0,01 % à 10 % en poids, par rapport au poids total de la composition, de préférence allant de 0,1 % à 5 % en poids, et préférentiellement allant de 0,1 % à 3 % en poids.
